# EUROPEAN PATENT SPECIFICATION

(11) **EP 0 695 168 B1**
(45) Date of publication and mention of the grant of the patent: **11.12.1996**
(21) Application number: 94912516.5
(22) Date of filing: 23.03.1994
(51) Int. Cl.: A61K 7/50

(54) **SURFACTANT COMPOSITION CONTAINING A SODIUM POLYOXYETHYLENE ALKYL ETHER SULFATE SURFACTANT AND A SULPHOSUCCINATE SURFACTANT**
TENSIDE ZUSAMMENSETZUNG ENTHALTEND EIN NATRIUM POLYEXYETHYLIERTES ALKYL ETHER SULFAT TENSID UND EIN SULFABENSTEINSÄURE TENSID
COMPOSITION D'AGENT TENSIOACTIF CONTENANT UN AGENT TENSIOACTIF AU SULFATE D'ETHER D'ALKYLE DE POLYOXYETHYLENE DE SODIUM ET UN AGENT AU SULFOSUCCINATE

(30) Priority: 08.04.1993 GB 9307449
(43) Date of publication of application: 07.02.1996
(73) Proprietor: UNILEVER PLC, London EC4P 4BQ (GB); UNILEVER N.V., 3013 AL Rotterdam (NL)
(72) Inventor: BENNER, Gerhard, D-21643 Beckdorf (DE)
(74) Representative: Elliott, Peter William
(86) International application number: EP9400920
(87) International publication number: WO9423695

(56) References cited:
- EP-A- 0 236 677
- EP-A- 0 531 650
- WO-A-92/04010
- WO-A-92/17154
- WO-A-92/18470
- CHEMICAL ABSTRACTS, vol. 112, no. 16, 16 April 1990, Columbus, Ohio, US; abstract no. 145339, BARANOWSKA B. ET AL 'Detergent composition exibiting hair conditionning action'
- CHEMICAL ABSTRACTS, vol. 118, no. 2, 11 January 1993, Columbus, Ohio, US; abstract no. 11515, KONUKI M. 'Bath preparation containing polyoxyethylene derivatives'

## Description

This invention relates to clear surfactant compositions. More particularly, it relates to clear surfactant compositions which may form the basis for personal cleansing compositions, such as shower gels.

Clear surfactant compositions are well known. For example, in GB1303810 (Unilever) there are described pourable liquid compositions for cleaning or rinsing which are clear. These compositions preferably comprise an anionic surfactant base, and preferably also a suspending agent such as a swelling clay or a soluble gum. They also comprise a visually distinct component of particle size at least 0.5mm in diameter, which is stably dispersed and suspended in the clear medium. The distinct component may comprise for example an emollient oil in the context of personal cleansing compositions. The distinct component is preferably encapsulated, or dispersed in a wax which breaks down during use.

Clear hair conditioning shampoo compositions comprising a monoester of sulfosuccinic acid, an ethoxylated aliphatic alcohol, water and optionally an amidopropyldimethylbetaine are known from Chemical Abstracts 112: 145339.

Additionally, surfactant compositions are known as for example described in CA 968248, which comprise an aqueous liquid or gel detergent composition containing at least 1% by weight of an electrolyte and containing capsules which are otherwise stable, but which release their contents when the composition is diluted with water. Such capsules are suspended in the liquid or gel such that they do not lose their spacial arrangement during storage, i.e. they are "spatially stable". The capsules consist essentially of a water soluble polymeric wall material which is gellable to a continuous matrix, and either encapsulates a continuous core of liquid or solid, or forms a homogenous mixture with the core material.

Also known, for example from EP 531 650, are surfactant compositions containing specific combinations of surfactants such as sodium lauryl ether sulphate and lauryl ether sulphosuccinates.

Chemical Abstracts 118: 11515 also describes surfactant compositions ethoxylated sulphosuccinates in combination with sodium lauryl ether sulphate.

The combination of sulphosuccinates and anionic surfactants such as sodium lauryl ether sulphate, together with fatty ether carboxylates is known from EP 236 677.

According to WO 92/04010 fatty alkyl sulphosuccinates are added to lipsomes contained in aqueous compositions to produce a stabilised lipsome suspension which can then be included in a detergent formulation containing, for example, sodium lauryl sulphate.

The addition of thickening agents and emulsifiers to surfactant containing compositions is known from WO 92/18470 and WO 92/17154.

Surprisingly, we have found that it is possible, using a carefully selected combination of surfactants to provide a surprisingly clear, almost transparent surfactant composition which not only has excellent surfactant and aesthetic properties in its own right, but has also a sufficiently good rheological structure so as to be capable of supporting suspended particles. Such a surfactant composition may form an excellent base for any gel-like personal cleansing composition, for example a shower gel or a shampoo.

Thus, according to the invention, there is provided an aqueous surfactant composition suitable for topical application to the human skin, comprising a sodium alkyl ether sulphate surfactant having from 0 to 5 ethylene oxide groups, a sulphosuccinate surfactant and a mixture of thickening agents selected from polysaccharide gums and fatty acid glyceride polyglycol ethers.

Preferably, the sodium alkyl ether sulphate surfactant is a sodium lauryl ether sulphate surfactant comprising an average of 2 to 4 ethylene oxide groups, in particular 3 ethylene oxide groups. Preferably the sodium alkyl ether surfactant is present in the composition at a level of 3-15% by weight.

According to a preferred aspect of the invention, the composition comprises from 5-35% by weight in total of surfactant, most preferably from 15-30% by weight of surfactant.

The sulphosuccinate surfactant used in compositions according to the invention may be any sulphosuccinate-containing surfactant entity. A preferred sulphosuccinate surfactant is Rewopol SBFA 30, ex Rewo, which is a 40% solution of disodium laurethsulphosuccinate. Preferably the composition comprises 3-10% by weight of the sulphosuccinate surfactant.

In a strongly preferred embodiment of the invention, the composition is a shower gel or shampoo composition and comprises one or more thickening agents. A preferred combination of thickening agents in compositions according to the invention is a polysaccharide gum, in particular a xanthan gum, such as Keltrol (trademark), and a fatty acid glyceride polyglycol ether thickener, such as Rewoderm LIS 75 (ex Rewo).

Preferably, the total amount of thickening agents in the compositions according to the invention is of from 0.5 to 3% by weight, preferably 1 to 1.5% by weight.

It has also been found highly desirable to include in compositions according to the invention glycerin, at a level of from 1 to 12% by weight, more preferably 5 to 9.5% by weight. At these levels, the glycerin has been found to contribute to the clarity of the composition. The exact levels of glycerin employed in the composition, may be readily adjusted by the skilled man to optimise clarity of the composition. Other agents that may be used in compositions according to the invention to promote clarity are 1,2-propanediol, and sorbitol.

According to a further preferred aspect of the invention, the composition additionally comprises a suspended phase. This is possible because the nature and viscosity of the composition is such that suspended particles of less than about 3mm diameter will remain suspended in a spatially stable arrangement. The suspended phase may comprise solid particles such as high density mica (eg Timiron), and components of a high specific weight, or it may comprise liquid components which are retained in a solid matrix. The liquid components in the composition may be for example emollients, oils, or other commonly used liquid benefit agents. The solid matrix for retaining the liquid component may be a homogenous mixture of liquid component and solid matrix material, or the liquid component may be encapsulated in the matrix material. The liquid component of the suspended phase may conveniently be delivered by rupturing of the capsules during use.

The suspended phase may be made by techniques known in the art, and may have as a solid matrix material, for example gelatin, or a wax, which may be for example impregnated with materials such as gum arabic. The suspended phase may conveniently contain a cosmetic material such as an oil; a preferred oil is advocado oil. Other preferred materials for incorporation in the suspended phase in compositions according to the invention include lipophilic materials, and also skin conditioning agents.

A preferred suspended phase has a particle diameter in the range 50-3000µm, more preferably 1700-2200µm.

It has been found that compositions according to the invention are superior to conventional similar compositions in that they not only provide excellent suspension of the suspended phase in the composition, but also they provide an excellent degree of transparency.

In a preferred embodiment of the invention in which the composition additionally comprises a suspended phase, the composition also comprises an emulsifier. Preferred emulsifiers according to the invention are PEG40 - hydrogenated castor oil emulsifiers, such as Cremophor RH40 or Cremophore RH410, ex. BASF. A further category of emulsifiers which may be used in compositions according to the invention are ethoxylated fatty acid derivatives. The purpose of this emulsifier is to prevent separation of the surfactant composition in the event that any oil escapes from the suspended phase. Such an emulsifier may be present in the composition at a level of 0.1 to 5% by weight.

Compositions according to the invention may also comprise other components commonly found in such compositions, including for example salts, emollients, oils, buffers, perfumes, antioxidants, preservatives, or pigments.

### Examples

The invention will now be illustrated with reference to the accompanying examples.

### Example 1

A shower gel was prepared according to the following composition.

| Component | Formulation % w/w |
|---|---|
| Genapol ZRO (1) | 14.5 |
| REWOPOL SBFA 30PH (2) | 13.3 |
| REWODERM LIS75 (3) | 0.8 |
| KELTROL RD (4) | 0.9 |
| Citric Acid | 0.3 |
| Vitamin E Acetate (5) | 0.1 |
| Sodium Chloride | 3.9 |
| Glycerin (6) | 11.0 |
| CREMOPHOR RH40 (7) | 0.5 |
| Microcapsules (8) | 0.5 |
| Potassium Sorbate | 0.5 |
| Perfume | 0.9 |
| Water | to 100 |

| | |
|---|---|
| (1) A 69% w/w solution of sodium lauryl ether sulphate surfactant, ex Hoechst. | |
| (2) A 40% solution of disodium laurethsulphosuccinate, containing Phenonip preservative, ex REWO. | |
| (3) A PEG fatty acid glyceride thickener, ex REWO. | |
| (4) Xanthan gum, ex KELCO. | |
| (5) Ex BASF. | |
| (6) 86% aqueous solution, ex Schrödinger/Unichema. | |
| (7) PEG-40 derivative of hydrogenated castor oil, ex BASF. | |
| (8) HC601, ex RAHN. The microcapsules comprise microencapsulated advocado oil and 2% Timiron Super Silver pigment, as a 60% slurry in water, encapsulated in gelatin/gum arabic, with Germaben II at 1% w/w in the aqueous phase as a preservative. Average particle diameter 2000µm. | |

The composition of example 1 was made according to the following method.

The Rewopol SBFA, Rewoderm LIS 75 and Genapol ZRO components are combined and heated to 60°C to dissolve the Rewoderm. To this warm solution is added the Keltrol, and subsequently 1% by weight of the sodium chloride to reduce any turbidity of the solution which arises after the Keltrol.

The remaining components of the composition can then be introduced carefully with stirring into the warm solution.

Finally, the viscosity of the composition can be modified by adjusting the amount of salt added to the composition, though the addition of too much salt can result in increased turbidity of the composition.

### Examples 2-9

Further compositions were prepared according to the method described in example one (all percentages are by weight of the composition). All provided shower gel formulations with a good degree of clarity and the ability to suspend microcapsules.

| **Example** | **2** | **3** | **4** | **5** | **6** | **7** | **8** | **9** |
|---|---|---|---|---|---|---|---|---|
| SLES3EO (1) | 14.5% | 14.5% | 14.5% | 14.5% | 14.5% | 14.5% | 14.5% | 14.5% |
| REWOPOL SBFA PH(2) | 13.3% | 13.3% | 13.3% | 13.3% | 13.3% | 13.3% | 13.3% | 13.3% |
| REWODERM LIS 75 (3) | 0.8% | 0.5% | 0.5 | 0.6 | 0.7 | 0.6 | 0.7 | 0.8 |
| KELTROL RD (4) | 0.6% | 0.6% | 0.6% | 0.6% | 0.8% | 0.6% | 0.8% | 0.9% |
| Citric Acid | 0.24% | 0.27% | 0.25% | 0.25% | 0.28% | 0.25% | 0.28% | 0.26% |
| Vitamin E Acelate (5) | 0.1% | 0.1% | 0.1% | 0.1% | 0.1% | 0.1% | 0.1% | 0.1% |
| Sodium Chloride | 4.4% | 4.9% | 4.9% | 3.9% | 3.7% | 3.9% | 3.7% | 3.9% |
| Glycerin (6) | 6.0% | - | - | 1.5% | 5.0% | 1.5% | 5.0% | 11.0% |
| 1,2 Propandiol | 2.0% | 2.0% | 2.0% | 1.0% | 1.0% | 1.0% | 1.0% | - |
| CREMOPHOR RH40 (7) | 0.5% | 0.5% | 0.5% | 0.5% | 0.5% | 0.5% | 0.5% | 0.5% |
| Micro-capsules (8) | - | 0.5% | - | 0.5% | 0.5% | - | - | 0.5% |
| Potassium Sorbate | 0.5% | 0.5% | 0.5% | 0.5% | 0.5% | 0.5% | 0.5% | 0.5% |
| Perfume | 0.9% | 0.9% | 0.9% | 0.9% | 0.9% | 0.9% | 0.9% | 0.9% |
| Water | to 100% | to 100% | to 100% | to 100% | to 100% | to 100% | to 100% | to 100% |
| (1)-(8) refer to the notes to the formulation in example 1. | | | | | | | | |

### Comparative Examples

Examples of similar shower gel compositions to example 1 were made up, only containing as a surfactant mixture sodium lauryl ether sulphate (SLES) and cocoa amido propyl betaine as opposed to SLES and sulphosuccinate. Such comparative compositions were found to be less clear than those containing SLES and sulphosuccinate.

Comparative compositions were also made up containing the same surfactant system in example 1, and with as an alternative thickening agent Carbopol, as opposed to Keltrol. Whilst the Carbopol comparative example provided a sufficiently stable matrix for suspending the microspheres, the composition had inferior clarity compared to that having Keltrol as a thickening agent.

Further compositions were prepared in which the Rewoderm LIS is replaced by Rewopal PEG 6000DS (PEG-150 distearate). These compositions however, provided compositions with inferior viscosity properties.

Compositions according to the invention additionally have satisfactory storage properties.

## Claims

1. An aqueous surfactant composition suitable for topical application to the human skin comprising a sodium alkyl ether surfactant having from 0 to 5 ethylene oxide groups, and a sulphosuccinate surfactant characterised in that it further comprises a mixture of thickening agents selected from polysaccharide gums and fatty acid glyceride polyglycol ethers.

2. A composition according to claim 1 wherein the sodium alkyl ether surfactant is present in the composition at a level of 3-15% by weight of the composition.

3. A composition according to claim 1 wherein the sodium alkyl ether surfactant is a sodium lauryl ether surfactant having an average of 3 ethylene oxide units.

4. A composition according to claim 1 wherein the sulphosuccinate surfactant is present at a level of from 3-10% by weight of the composition.

5. A composition according to claim 1, comprising a suspended phase.

6. A composition according to claim 1, comprising glycerin at a level of from 1 to 10% by weight of the composition.

7. A composition according to claim 1 wherein the polysaccharide gum is xanthan gum.

8. A composition according to claim 1, wherein the composition additionally comprises an emulsifier comprising a PEG 40-hydrogenated castor oil emulsifier and/or an ethoxylated fatty acid derivative emulsifier.

9. A shampoo or shower gel composition suitable for topical application to the human skin comprising 3-15% by weight of a sodium lauryl ether surfactant having from 0 to 5 ethylene oxide groups, from 3-10% by weight of a sulphosuccinate surfactant, from 0.5 to 3% of a mixture of thickening agents comprising a polysaccharide gum and a fatty acid glyceride polyglycol ether thickener, from 1 to 10% by weight of glycerin and from 0.1 to 5% by weight of an emulsifier comprising a PEG40 hydrogenated castor oil emulsifier.

## Patentansprüche

1. Wässerige Tensidzusammensetzung, geeignet zur örtlichen Anwendung auf der menschlichen Haut, umfassend ein Natriumalkylethertensid mit 0 bis 5 Ethylenoxidgruppen und ein Sulfosuccinattensid, dadurch gekennzeichnet, daß es weiterhin ein Gemisch aus Verdickungsmitteln, ausgewählt aus Polysaccharidgummen und Fettsäureglyceridpolyglycolethern, umfaßt.

2. Zusammensetzung nach Anspruch 1, wobei das Natriumalkylethertensid in der Zusammensetzung in einem Ausmaß von 3-15 Gewichtsprozent der Zusammensetzung vorliegt.

3. Zusammensetzung nach Anspruch 1, wobei das Natriumalkylethertensid ein Natriumlaurylethertensid mit im Durchschnitt 3 Ethylenoxideinheiten ist.

4. Zusammensetzung nach Anspruch 1, wobei das Sulfosuccinattensid in einem Ausmaß von 3-10 Gewichtsprozent der Zusammensetzung vorliegt.

5. Zusammensetzung nach Anspruch 1, umfassend eine suspendierte Phase.

6. Zusammensetzung nach Anspruch 1, umfassend Glyzerin in einem Ausmaß von 1 bis 10 Gewichtsprozent der Zusammensetzung.

7. Zusammensetzung nach Anspruch 1, wobei das Polysaccharidgummi Xanthangummi ist.

8. Zusammensetzung nach Anspruch 1, wobei die Zusammensetzung außerdem einen Emulgator umfaßt, der einen Emulgator aus PEG 40-hydriertem Rizinusöl und/oder einen Emulgator aus ethoxyliertem Fettsäurederivat umfaßt.

9. Shampoo oder Duschgelzusammensetzung, geeignet zur örtlichen Anwendung auf der menschlichen Haut, umfassend 3-15 Gewichtsprozent eines Natriumlaurylethertensids mit 0 bis 5 Ethylenoxidgruppen, 3-10 Gewichtsprozent eines Sulfosuccinattensids, 0,5 bis 3% eines Gemisches von Verdickungsmitteln, umfassend ein Polysaccharidgummi und ein Fettsaureglyceridpolyglycolether-Verdickungsmittel, 1 bis 10 Gewichtsprozent Glyzerin und 0,1 bis 5 Gewichtsprozent eines Emulgators, der einen Emulgator aus PEG 40-hydriertem Rizinusöl umfaßt.

## Revendications

1. Composition tensioactive aqueuse appropriée pour l'application locale à la peau humaine comprenant un tensioactif alkyléther sodique ayant de 0 à 5 groupes oxyde d'éthylène et un tensioactif sulfosuccinate, caractérisée en ce qu'elle comprend en plus un mélange d'agents épaississants choisis parmi les gommes polysaccharide et les glycéride-polyglycoléthers d'acides gras.

2. Composition selon la revendication 1, dans laquelle le tensioactif alkyléther sodique est présent dans la composition en une proportion de 3 à 15% en poids de la composition.

3. Composition selon la revendication 1, dans laquelle le tensioactif alkyléther sodique est un tensioactif lauryléther sodique ayant une moyenne de 3 motifs d'oxyde d'éthylène.

4. Composition selon la revendication 1, dans laquelle le tensioactif sulfosuccinate est présent en une proportion de 3 à 10% en poids de la composition.

5. Composition selon la revendication 1, comprenant une phase mise en suspension.

6. Composition selon la revendication 1, comprenant de la glycérine en une proportion de 1 à 10% en poids de la composition.

7. Composition selon la revendication 1, dans laquelle la gomme polysaccharide est la gomme de xanthane.

8. Composition selon la revendication 1, dans laquelle la composition comprend en outre un émulsifiant comprenant un émulsifiant huile de ricin hydrogénée/PEG-40 et/ou un émulsifiant dérive d'un acide gras éthoxylé.

9. Composition de shampooing ou de gel de douche appropriée pour l'application locale à la peau humaine comprenant 3 a 15% en poids d'un tensioactif lauryléther sodique ayant de 0 à 5 groupes oxyde d'éthylène, de 3 à 10% en poids d'un tensioactif sulfosuccinate, de 0,5 a 3% d'un mélange d'agents épaississants comprenant une gomme polysaccharide et un épaississant glycéridepolyglycol-éther d'acide gras, de 1 à 10% en poids de glycérine et de 0,1 à 5% en poids d'un émulsifiant comprenant un émulsifiant huile de ricin hydrogénée/PEG 40.
